# EUROPEAN PATENT SPECIFICATION

(11) **EP 2 327 676 B1**
(45) Date of publication and mention of the grant of the patent: **05.03.2014**
(21) Application number: 09177215.2
(22) Date of filing: 26.11.2009
(51) Int. Cl.: C07B 31/00

(54) **Reaction of organic compounds with low amounts of hydrogen**
Reaktion organischer Verbindungen mit niedrigen Mengen von Wasserstoff
Réaction de composés organics avec de faibles quantités d'hydrogène

(43) Date of publication of application: 01.06.2011
(73) Proprietor: SANDOZ AG, 4056 Basel (CH)
(72) Inventor: Decristoforo, Martin, 6250 Kundl (AT)
(74) Representative: Wichmann, Hendrik

(56) References cited:
- US-A- 3 859 377
- US-A- 5 504 268

## Description

### FIELD OF THE INVENTION

The present invention relates to a process for the reaction of a compound as defined in the claims with hydrogen wherein the reaction is conducted using a hydrogen-containing gas comprising up to 10 vol.% hydrogen and at least 90 vol.% of an inert gas and wherein the compound to be reacted with hydrogen is provided in a liquid phase. The present invention refers to hydrogenation and hydrogenolysis reactions.

### BACKGROUND OF THE INVENTION

The hydrogenation reactions are commonly employed in order to reduce compounds containing a double or triple bond. The sources of hydrogen vary depending on the type and scale of the reaction involved. While gaseous hydrogen in often used on an industrial scale, transfer hydrogenations using hydrogen donors such as hydrazine can be used in special applications.

In hydrogenolysis reactions a compound containing a carbon-carbon or carbon-heteroatom single bond is reacted with hydrogen whereby the carbon-carbon or carbon-heteroatom single bond is cleaved. Hydrogenolysis is used on a large scale for desulfurization in petroleum refining. It is also used commercially among others to prepare alcohols from the corresponding esters or to remove protecting groups like benzylesters, p-nitrobenzylesters benzhydrylesters etc.

CN-A-1569783 describes a non-petroleum route process for preparing ethylene using a gas mixture of pure acetylene, hydrogen and nitrogen as the raw material gas, wherein the volume content of acetylene in the raw material reaction gas is 10 to 40 %.

US 5504268 (A) describes a process for the selective hydrogenation of aromatic acetylene compounds present as impurities in vinyl-aromatic compounds comprising adding hydrogen and an inert gas to a liquid phase vinyl-aromatic compound containing an aromatic acetylene compound and contacting the aromatic acetylene compound with hydrogen in the presence of a selective hydrogenation catalyst, wherein the partial hydrogen pressure is from about 0.001 to about 0.05 bar.

US 3859377 (A) describes a process for the selective hydrogenation of C4 acetylenes in admixture with butadiene in the liquid phase which comprises reacting said C4 hydrocarbon mixture with a stream of hydrogen diluted to not more than 50 mole percent hydrogen in inert gas in concurrent upflow reaction over a catalyst comprising from 0.01 to 1.0 weight percent palladium impregnated to a depth of at least 0.12 inch (0.3 cm) on a kieselguhr support having a surface area of from 0.5 to 20 m²/g and a volume of macropores greater than 700 A in diameter of at least 75% of total pore volume of said support at a temperature of from 50° to 175°F (10°C to 80°C) and under a pressure sufficient to maintain the C4 hydrocarbon.

At present, the reactions using hydrogen gas are typically conducted with pure hydrogen gas. Because the employed gas is explosive or forms explosive mixtures together with air, strict safety measures have to be taken. These safety measures make the reactions with hydrogen complicated and costly.

It is an object of the present invention to provide an improved process which is more simple and/or less costly than previous processes. A further object of the present invention is to provide a process which can be applied in large scale applications. Yet another object of the present invention is to provide a process which does not require the usual strict safety measures, e.g. protective measures against combustion and/or explosion usually required for catalytic hydrogenation reactions.

### SUMMARY OF THE INVENTION

The present invention relates to a process for the reaction of a compound as defined in the claims with hydrogen wherein the reaction is conducted using a hydrogen-containing gas comprising up to 10 vol.% hydrogen and at least 90 vol.% of an inert gas and wherein the compound to be reacted with hydrogen is provided in a liquid phase.

### BRIEF DESCRIPTION OF THE DRAWINGS

- Figure 1: shows the ¹H-NMR-spectrum of the product of Example 1.
- Figure 2: shows the ¹H-NMR-spectrum of the product of Example 2.

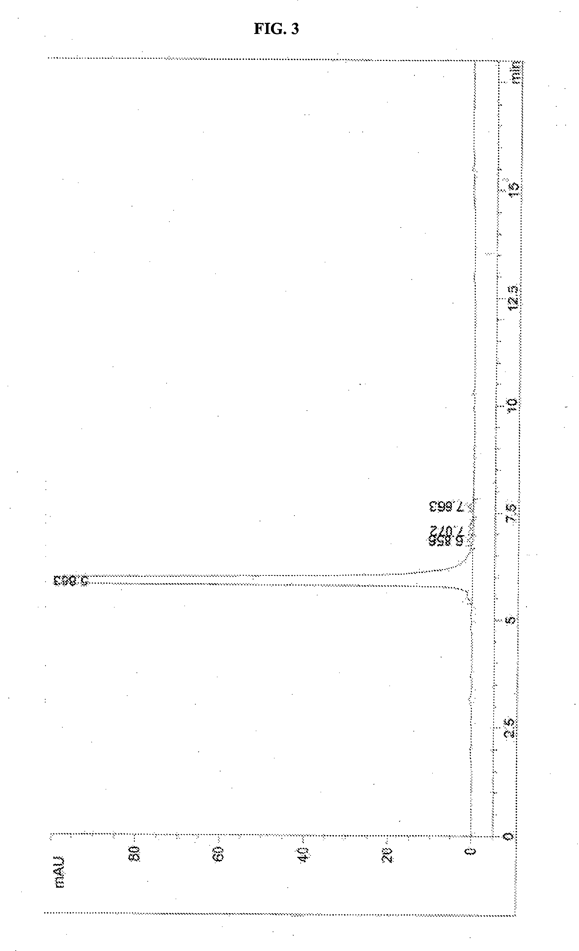

- Figure 3: shows the HPLC-chromatogram of the product of Example 3.

### DETAILED DESCRIPTION OF THE INVENTION

The present invention relates to a process for the reaction of a compound as defined in the claims with hydrogen wherein the reaction is conducted using a hydrogen-containing gas comprising up to 10 vol.% hydrogen and at least 90 vol.% of an inert gas and wherein the compound to be reacted with hydrogen is provided in a liquid phase.

The present invention refers to hydrogenation reactions and hydrogenolysis reactions.

A hydrogenation reaction is defined as a reaction in which hydrogen (H₂) is reacted with a compound containing a double or triple bond and the hydrogen is added to the double or triple bond of the compound. In this reaction hydrogen is added without cleaving the linkage between the atoms connected by the double or triple bond. The resultant product corresponds to the initial compound but, depending on the employed hydrogenation reaction, has a single or double bond. The term "hydrogenation reaction" refers to the above mentioned reaction and, unless stated otherwise, does not include the step in which a catalyst is regenerated.

A hydrogenation reaction is shown schematically in the following scheme whereby atoms are denoted by ★:

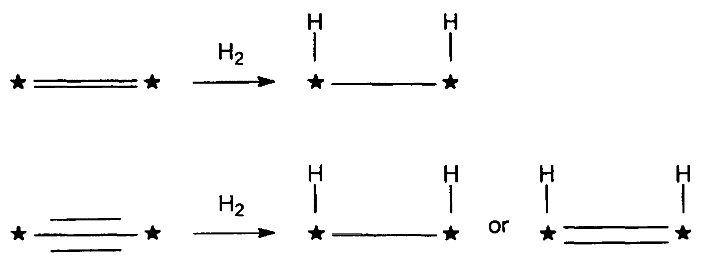

Many types of hydrogenation reactions are known in the art. The process of the present invention can be applied to all known hydrogenation reactions as defined in the claims in which hydrogen gas is employed as the hydrogen source. A review over possible hydrogenation reactions which can be used in the present invention can be found in "Advanced Organic Chemistry • Part B: Reactions and Synthesis", Chapter 5, 5th edition, Francis A. Carey, Richard J. Sundberg, Springer Verlag, 2007, and M. Freifelder, "Catalytic hydrogenation in Organic Synthesis: Procedures and Commentary, Wiley-Interscience, New York, 1978.

A hydrogenolysis reaction is defined as a reaction in which a compound containing a carbon-carbon or carbon-heteroatom single bond is reacted with hydrogen whereby the carbon-carbon or carbon-heteroatom single bond is cleaved.

A hydrogenolysis reaction is shown schematically in the following scheme whereby atoms are denoted by * :

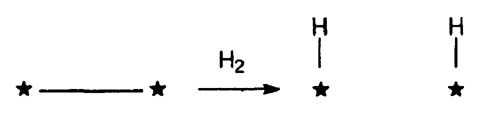

Many types of hydrogenolysis reactions are known in the art. The process of the present invention can be applied to all known hydrogenolysis reactions as defined in the claims in which hydrogen gas is employed as the hydrogen source. A review over possible hydrogenolysis reactions which can be used in the present invention can be found in "Advanced Organic Chemistry • Part B: Reactions and Synthesis", Chapter 5, 5th edition, Francis A. Carey, Richard J. Sundberg, Springer Verlag, 2007; and M. Freifelder: "Catalytic hydrogenation in Organic Synthesis: Procedures and Commentary", Wiley-Interscience, New York, 1978.

The reaction of the present invention is conducted in the liquid phase. If the compound to be reacted with hydrogen is liquid, the liquid phase can be or can comprise the compound per se. Alternatively, the liquid phase can comprise a solution, suspension or emulsion of the compound which is to be reacted with hydrogen.

The liquid phase can be selected from any liquid which is suitable for the specific reaction which is to be conducted. Examples of typical solvents which can be used in the liquid phase include polar solvents such as water, alcohols (such as C₁₋₄ alcohols), esters (such as ethyl acetate, which can be used under gentle conditions known in the art), ethers (such as dioxane or THF, which can be used under gentle conditions, such as room temperature and atmospheric pressure), alkanes (such as cyclohexane) and organic acids (such as acetic acid).

While high amounts of hydrogen are typically employed in gas phase reactions, it has been surprisingly found that the process of the present invention can be conducted with low amounts of hydrogen in the hydrogen-containing gas which is passed, e.g. bubbled, through the liquid reaction medium. Without wishing to be bound by theory, it is assumed that the hydrogen in the hydrogen-containing gas becomes sufficiently dissolved in the liquid reaction medium or, if a catalyst is employed, can sufficiently interact with the catalyst even if very low amounts of hydrogen are present in the hydrogen-containing reaction gas mixture.

The process of the present invention could be conducted without a catalyst. However, a catalyst is typically desirable because the reaction with hydrogen can proceed under much milder conditions. The catalyst, if present, is typically either a homogeneous or heterogeneous catalyst, preferably a heterogeneous catalyst.

Homogeneous catalysts are soluble in the reaction medium. Examples of possible homogeneous catalysts include soluble complexes of transition metals. Examples of suitable transition metals include platinum group metals (such as Pd, Pt, Ru, Ir and Rh) as well as iron, cobalt, and nickel. Particular examples of possible homogeneous catalysts can be found in "Advanced Organic Chemistry Part B: Reactions and Synthesis", Chapter 5, 5th edition, Francis A. Carey, Richard J. Sundberg, Springer Verlag, 2007 and M. Freifelder: "Catalytic hydrogenation in Organic Synthesis: Procedures and Commentary", Wiley-Interscience, New York, 1978.

Heterogeneous catalysts are not soluble in the reaction medium. Examples of possible heterogeneous catalysts are solid transition metals or their compounds, typically in a finely divided form, or transition metals or their compounds disposed on a support. Examples of suitable transition metals include platinum group metals (such as Pd, Pt, Ru, Ir and Rh) as well as iron, cobalt, and nickel. Chromite catalysts are further examples of possible heterogeneous catalysts. Carbon, calcium carbonate, barium sulfate, alumina and silica can be given as examples of possible supports. Examples of possible heterogeneous catalysts include Raney nickel, chromite catalysts, as well as platinum group metals on a support (e.g., platinum group metal on carbon such as platinum or palladium on carbon) or a platinum group metal as sponge or as oxide e.g. platinum dioxide (Adams catalyst). Particular examples of possible heterogeneous catalysts can be found in "Advanced Organic Chemistry Part B: Reactions and Synthesis", Chapter 5, 5th edition, Francis A. Carey, Richard J. Sundberg, Springer Verlag, 2007 and M. Freifelder: "Catalytic hydrogenation in Organic Synthesis: Procedures and Commentary", Wiley-Interscience, New York, 1978.

The reaction can be conducted at any suitable pressure. The pressure will depend on the specific reaction which is to be conducted. Typically the reaction will be conducted at atmospheric pressure or elevated pressure. The pressure can, e.g., range from about 1 x 10⁵ Pa to about 3.5 x 10**⁷** Pa. In one embodiment the pressure is about atmospheric pressure (about 1 x 10⁵ Pa). In another embodiment the pressure is about 1 x 10⁵ Pa to about 7 x 10⁵ Pa. In a further embodiment the pressure is about 7 x 10⁵ Pa to about 3.5 x10⁷ Pa. The above values for gas pressure relate to the total pressure of the gas to be used in the hydrogenation reaction, not to the partial hydrogen pressure.

The reaction can be conducted at any suitable temperature. The temperature will depend on the specific reaction which is to be conducted. Typically the reaction will be conducted at room temperature (e.g., about 20 °C to about 25 °C) or at elevated temperature. The temperature can, e.g., range from about -25 °C to about 300 °C, depending on the specific reaction to be conducted. In one embodiment the temperature is preferably from about -25 °C to about 250°C, alternatively from about -25 °C to about 100°C and more preferably from about 0°C to about 50°C.

Known additives and auxiliaries can be employed in the process of the present invention, as occasion requires. Examples are desactivating substances to influence the reactivity of the catalyst, for example lead as used for palladium on calcium carbonate catalysts, e.g. as detailed in Lindlar, H.; Dubuis, R. (1973), "Palladium Catalyst for Partial Reduction of Acetylenes", Org. Synth., Coll. Vol. 5: 880. Catalysts with modified reactivity are, for example, employed for the partial reduction of carbon-carbon triple bonds to carbon-carbon double bonds and for the reduction of acid chlorides to aldehydes.

The process of the present invention can be conducted in a batch or continuous manner. In a preferred embodiment, it is conducted by continuously flowing the hydrogen-containing gas through the liquid phase. In a preferred embodiment, the gas is simply bubbled through the reaction liquid. Alternatively, the gas can be injected by means of a jet or by means of a sintered metal or glass candle. The gas also can be superimposed over the liquid in an autoclave at elevated pressure, in this case it is to be changed several times until the reaction is finished.

The hydrogen-containing gas comprises up to 10 vol.% hydrogen and at least 90 vol.% of an inert gas. A skilled person will be able to determine the lower limit of hydrogen which is suitable for the reaction which is to be conducted by way of a simple series of experiments. For instance, he could start with an initial amount of 5 vol.% hydrogen and reduce the amount of hydrogen in the hydrogen-containing gas in a stepwise manner and observe, whether the desired product resulting from hydrogenation or hydrogenolysis still forms.

Surprisingly, the present inventors have discovered that the overall reaction conditions for the process of the invention remain essentially the same with regard to temperature and pressure as compared to the corresponding process which uses pure hydrogen. This means that a compound which can be reacted with hydrogen in a liquid reaction medium at room temperature and under ambient pressure using pure hydrogen as the reaction gas can also be reacted in the same liquid reaction medium at room temperature and under ambient pressure using the reaction gas mixtures used in the process of the present invention. Thus, the skilled person can start from the ample knowledge about reactions with hydrogen which employ pure hydrogen as a reaction gas and can use these conditions as a starting point by replacing a gas containing 100 vol.% hydrogen by the reaction gas mixtures used in the process of the present invention.

The process of the invention is preferably conducted using a gas comprising about 0.1 to about 10 vol.% hydrogen and about 90 to about 99.9 vol.% of an inert gas. In a preferred embodiment the gas comprises about 1 to about 7 vol.% hydrogen and about 93 to about 99 vol.% of an inert gas, more preferably the gas comprises about 2 to about 6 vol.% hydrogen and about 94 to about 98 vol.% of an inert gas, most preferably about 5 vol.% hydrogen and about 95 vol.% of an inert gas. The commercially available mixture which consists of about 5 vol.% hydrogen / 95 vol.% nitrogen is particularly preferred in the process of the invention.

In a preferred embodiment the gas consists essentially of the above indicated amounts of hydrogen and the inert gas. In this context "consists essentially of" refers to a gas which can include up to about 5 vol.%, preferably up to about 2 vol.%, more preferably up to about 1 vol.%, components other than hydrogen and the inert gas. In a further preferred embodiment the gas consists of above indicated amounts of hydrogen and the inert gas.

The inert gas can be any gas which is inert in the reaction at issue. Examples of inert gases include nitrogen and noble gases (such as argon) as well as mixtures thereof. In view of its cost, nitrogen is the preferred inert gas.

By employing the above described hydrogen-containing gas, the present invention provides a simple, cost effective and safe method for conducting reactions with hydrogen. Because the gas is not explosive either alone or in combination with air, it is possible to avoid the strict safety measures which were previously required for reactions with pure hydrogen. This enables the skilled person to use equipment for reactions with hydrogen which would have previously been considered unsuitable for this purpose due to lack of sufficient safety measures and/or to work in environments which would have previously been considered unsuitable for this purpose due to lack of sufficient safety measures.

The substrate (i.e., the compound to be reacted with hydrogen) is defined in the claims for the hydrogenation reaction and is any compound which is susceptible to the desired hydrogenolysis reaction. Preferably the compound is an organic compound, more preferably having a molecular weight from 28 Da to 100 kDa, even more preferably from 40 Da to 50 kDa, such as from 50 Da to 10 000 Da. In the case of a hydrogenation reaction the substrate is a compound containing a double or triple bond. The compound is typically an organic compound. In one embodiment the compound is non-polymeric.

The double or triple bond is preferably selected from the group consisting of

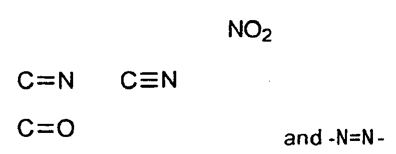

Examples of compounds including suitable double or triple bonds include ketones, aldehydes, nitro compounds, imines, oximes, nitriles, and heteroaryl compounds, hydrazones, azines and azo compounds, with ketones, aldehydes, esters, nitro compounds, imines, oximes and nitriles being preferred and alkenes, alkynes, nitro compounds, imines and oximes being even more preferred.

Typical hydrogenation reactions include the following:
(i) reduction of a nitro moiety to an amine moiety;
(ii) reduction of an imine moiety to an amine moiety;
(iii) reduction of an oxime moiety to an amine moiety; and
(iv) reduction of a nitrile group to an amine group;
(v) reduction of a ketone moiety to an alcohol moiety;
(vi) reduction of an aldehyde moiety to an alcohol moiety;
(vii) reduction of a heteroaryl moiety to the corresponding saturated hetero ring moiety.
(viii) reduction of an acid chloride moiety to the corresponding aldehyde (Rosenmund reduction)

Reactions (i) to (vi) are more preferred, reactions (i) to (iii) are even more preferred. In general, less harsh conditions can be employed for the more preferred reactions. In particular, the most preferred reactions work even at room temperature and ambient pressure to a slightly elevated pressure of not more than 7 * 10⁵ Pa. Suitable catalysts and/or reaction conditions for a particular substrate to be reacted with hydrogen can be found in "Advanced Organic Chemistry Part B: Reactions and Synthesis", Chapter 5, 5th edition, Francis A. Carey, Richard J. Sundberg, Springer Verlag, 2007 and M. Freifelder: "Catalytic hydrogenation in Organic Synthesis: Procedures and Commentary", Wiley-Interscience, New York, 1978.

One possible application of the embodiment in which a nitro moiety is reduced to an amine moiety is the hydrogenation of 9-nitrominocycline, for example during the preparation of tigecycline. This hydrogenation is typically conducted using a heterogeneous catalyst such as a catalyst based on Pd, Pt, Ir or Ni and proceeds quickly even at RT and about atmospheric pressure (about 1 x 10⁵ Pa). For suitable conditions see also Example 3.

One possible application of the embodiment in which a C=N moiety is reduced to an amine moiety is the hydrogenation of aprimin, for example, during the preparation of aprepitant. This hydrogenation is typically conducted using a heterogeneous catalyst such as a catalyst based on Pd, Pt, Ir or Ni.

Examples of possible homogeneous catalysts for hydrogenation reactions include Wilkinson's catalyst (Ph₃P)₃RhHal), Crabtree's catalyst ([(tris-cyclohexylphosphine) Ir (1,5-cyclooctadiene) (pyridine)] PF₆ ) and Brown's catalyst ([(Ph₂P(CH₂)₄PPh₂) Rh (nbd)]⁺ BF₄-). All of these catalysts can be employed in the present invention, for example, to hydrogenate alkenes.

If desired, the hydrogenation can be conducted in an enantioselective manner by using chiral catalysts. Examples of possible enantioselective catalysts include transition metal complexes with DIOP, CHIRAPHOS, PROPHOS, PHENPHOS, CYCPHOS, DBPP, NORPHOS, CAMPHOS, DPCP, PYRPHOS, BPPM, PPPFA, DUPHOS, DIPHEMP, BINAP, DIPAMP, and DINAP.

A further example of a possible hydrogenation reaction is the reaction with a Lindlar catalyst.

The above mentioned catalysts are given as examples of possible catalysts for hydrogenation reactions which can be used in the present invention. However, they serve as an illustration and should not be construed as a limitation of the present invention, which is not restricted thereto.

An example of the hydrogenation of a nitro moiety is provided in the below reaction scheme 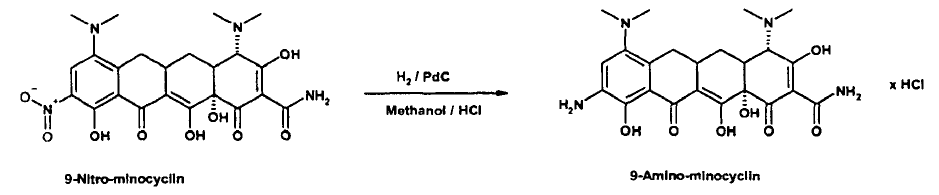 wherein the catalyst is 10% Palladium on charcoal, moistened with 50% of water and the process is carried out under the following conditions: A 3-5% solution of the substrate in methanol / hydrochloric acid is charged with an amount of catalyst corresponding to 15-20% w/w of the amount of substrate (on dry basis) and then a 5v% hydrogen /95v% nitrogen mixture is bubbled through the slurry at 20-25°C and at a slight overpressure of approx. 100 mbar until the starting material has disappeared, as detected by
HPLC.

An example of the hydrogenation of an olefin to a saturated hydrocarbon not according to the invention is provided in the below reaction scheme:

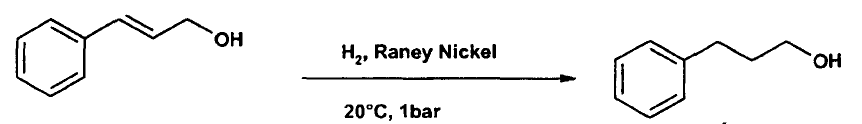

An example of the hydrogenation of an alkyn to a saturated hydrocarbon not according to the invention is provided in the below reaction scheme:

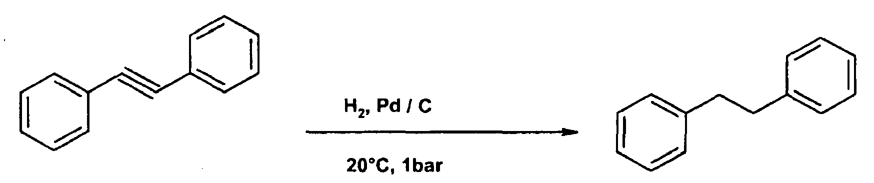

An example of the hydrogenation of a nitrile to a primary amine is provided in the below reaction scheme:

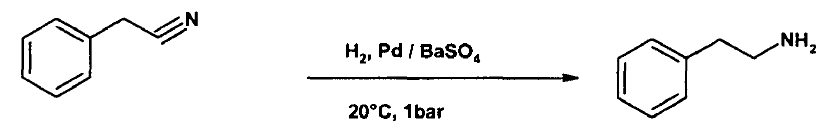

The skilled person will, however, appreciate that more typically the reduction of nitriles to primary amines requires elevated temperatures of between 50°C and 100°C and elevated pressure.

In a particularly preferred embodiment, the present invention relates to a process for the reaction of a compound as defined in the claims with hydrogen, wherein the reaction is a hydrogenation reaction and is conducted using a hydrogen-containing gas comprising about 1 vol.% to about 7 vol.% hydrogen and about 93 vol.% to about 99 vol.% of an inert gas, wherein the compound to be reacted with hydrogen is provided in a liquid phase, wherein the compound is an organic compound having a molecular weight from 50 Da to 10 000 Da, wherein the pressure is about 1 x 10⁵ Pa to about 7 x 10⁵ Pa, wherein the temperature is from about 0°C to about 50°C, wherein the substrate for the hydrogenation reaction is a compound containing a double or triple bond which is susceptible to cleavage under the above conditions of temperature and gas pressure, in particular wherein the reaction is selected from the group consisting of the reduction of a nitro moiety to an amine moiety, the reduction of an imine moiety to an amine moiety, and the reduction of an oxime moiety to an amine moiety.

In the case of a hydrogenolysis reaction the substrate is a compound containing a carbon-carbon or carbon-heteroatom single bond which is susceptible to cleavage in a reaction with hydrogen. The compound is typically an organic compound. In one embodiment the compound is non-polymeric. The compound preferably has a moiety selected from the group consisting of 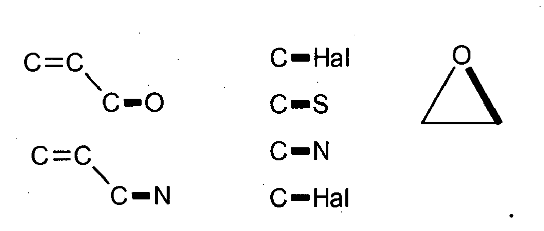 wherein the bond which is cleaved is indicated as a bold line.

Typical hydrogenolysis reactions include the following:
(i) removal of a benzyloxycarbonyl group by hydrogenolysis;
(ii) the reaction of a benzyl ester to a corresponding carboxylic acid and toluene;
(iii) the reaction of a benzyl ether to the corresponding benzyl compound and alcohol;
(iv) the reaction of a benzyldialkylamine to the corresponding diallcylamine and toluene;
(v) the reaction of a compound having a C-Hal bond to the corresponding compound having a C-H bond (wherein Hal is Cl, Br, I, or F; preferably I, Br or Cl; more preferably I or Br; even more preferably I);
(vi) the ring opening of an epoxide to the corresponding alcohol;
(vii) the cleavage of a C-S bond to result in a corresponding compound having a C-H bond and hydrogensulfide;
   and
(viii) the reaction of an ester to a corresponding primary alcohol.

Reactions (i) to (v) are preferred, reactions (i) to (iv) are more preferred and reactions (i) and (ii) are even more preferred.

In one preferred embodiment it is possible to employ the hydrogenolysis reaction according to the present invention to remove protecting groups. An example of this embodiment is the hydrogenolysis of an optionally substituted benzylether to an alcohol and the optionally substituted benzyl compound. 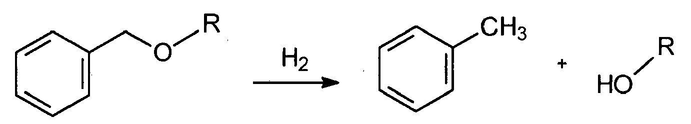 wherein the phenyl ring can be optionally substituted (e.g. by a methoxy or halogen) and wherein R is an residue compatible with the catalytic hydrogenation reaction under the particular conditions employed.

A further preferred example of a hydrogenolysis process of the invention for the removal of a protecting group is the cleavage of a benzyloxycarbonyl (Cbz) group. 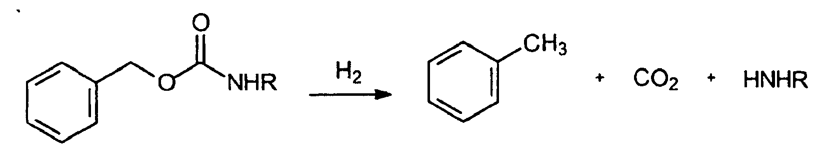 wherein the phenyl ring can be optionally substituted by a residue compatible with the catalytic hydrogenation reaction under the particular conditions employed (e.g. by alkyl, methoxy, halogen) and wherein R is an residue compatible with the catalytic hydrogenation reaction under the particular conditions employed. Cleavage of the benzyloxycarbonyl (Cbz) group is for example room temperature at about atmospheric pressure.

An example of another type of hydrogenolysis reaction includes the Rosenmund reduction, in which an acid chloride is reduced to the corresponding aldehyde with hydrogen in the presence of a partially desactivated palladium catalyst (desactivation with chinoline, sulfur compounds and the like).

In a particularly preferred embodiment, the present invention relates to a process for the reaction of a compound with hydrogen, wherein the reaction is a hydrogenolysis reaction and is conducted using a hydrogen-containing gas comprising about 1 vol.% to about 7 vol.% hydrogen and about 93 vol.% to about 99 vol.% of an inert gas, wherein the compound to be reacted with hydrogen is provided in a liquid phase, wherein the compound is an organic compound having a molecular weight from 50 Da to 10 000 Da, wherein the pressure is about 1 x 10⁵ Pa to about 7 x 10⁵ Pa, wherein the temperature is from about 0°C to about 50°C, in particular wherein the substrate for the hydrogenolysis reaction is a compound containing a carbon-carbon or carbon-heteroatom single bond which is susceptible to cleavage under the above conditions of temperature and gas pressure, in particular wherein the reaction is the removal of a benzyloxycarbonyl group.

The present invention also relates to the use of a hydrogen-containing gas comprising up to about 10 0 vol.% hydrogen and at least about 90 vol.% of an inert gas for the catalytic hydrogenation or hydrogenolysis of an organic compound susceptible to catalytic hydrogenation or hydrogenolysis, wherein the substrate for catalytic hydrogenation or hydrogenolysis is provided in a liquid phase, in particular to the uses resulting from the application of the above described processes of the present invention.

The above mentioned catalysts are given as examples of possible catalysts for hydrogenolysis reactions which can be used in the present invention. However, the present invention is not restricted thereto.

The invention will now be explained with the help of the following examples. However, these examples should not be construed so as to be in any way limiting to the scope of the present invention.

### EXAMPLES

### Comparative Example 1

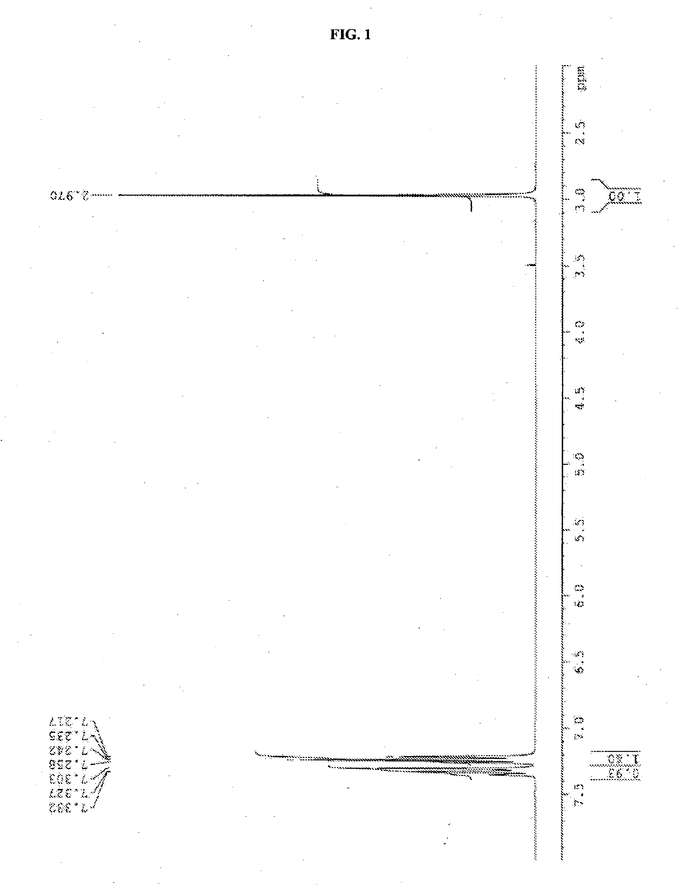

4.46 g diphenylacetylene were dissolved in 150 mL methanol. 1 g 10 % palladium on carbon (available as RD-9210 from Hindustan Platinum Inc) was added. A mixture of 95 vol.% N₂ and 5 vol.% H₂ (available from Linde Gas) was passed through the suspension for 11 hours at a flow rate of approx. 30 L/h at room temperature (20-25°C) and an overpressure of 100 mbar. The catalyst was removed by filtration. The solution was concentrated *in vacuo.* The resultant product was isolated by filtration and dried. 2.93 g diphenylethane were obtained.
¹H-NMR (CDCl₃, 300 MHz): 2.97 ppm (s), 4H, 2 x CH₂; 7.21-7,26 ppm (m), 6H, 2 x H3/4/5 arom.; 7.30-7.36 ppm (m), 4H, 2 x H2/6 arom.
The NMR-spectrum of the product is shown in Figure 1. Reduction of the alkyne to the alkane was essentially complete, with no detectable products from incomplete reduction of the triple bond to the alkene level, as can be taken from ratio of the integral for the alkane protons at 2.97ppm to the sum of the integrals for the aromatic protons at around 7.2 to 7.3 ppm on the one hand and the absence of a peak corresponding to olefinic protons (between 5ppm and 7ppm) on the other.

### Example 2

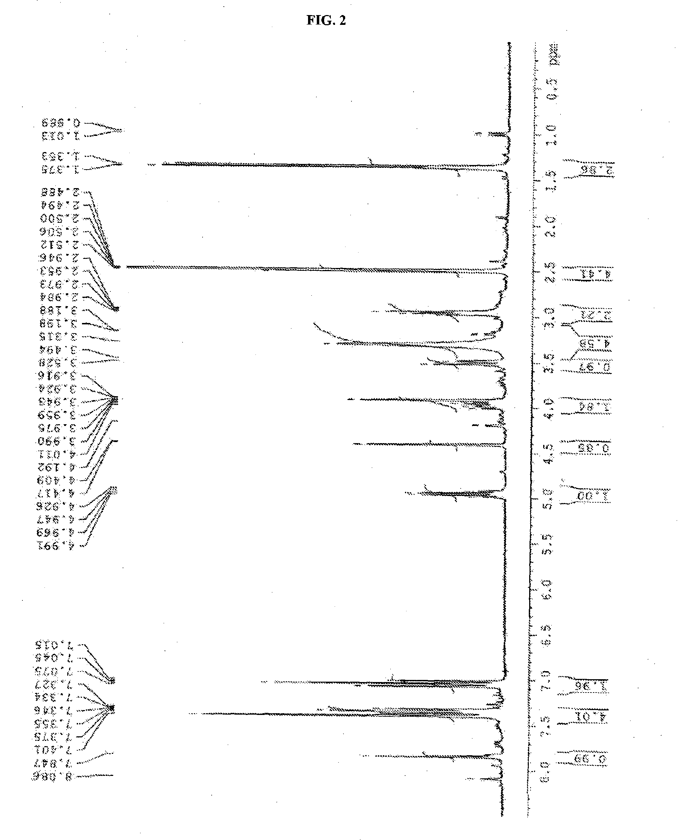

0.94 g of 2-[(1R)-1-[3,5-bis(trifluoromethyl)phenyl]ethoxy]-3-(4-fluorophenyl)-5,6-dihydro-2H-1,4-oxazine were dissolved in 150 mL methanol. 1 g 10 % palladium on carbon (available as RD-9210 0 from Hindustan Platinum Inc) was added. A mixture of 95 vol.% N₂ and 5 vol.% H₂ (available from Linde Gas) was passed through the suspension for 6.5 hours at a flow rate of 30 L/h. The temperature was 25 to 30 °C and the overpressure was approx. 150 mbar. The catalyst was removed by filtration. The solution was concentrated *in vacuo* until an oil was obtained. 0.9 g 2-[(1R)-1-[3,5-bis(trifluoromethyl)phenyl]ethoxy]-3-(4-fluorophenyl)-(2R,3S)-morpholine were obtained.
¹H-NMR (DMSO-d6, 300 MHz): 1.36 ppm (d, 3H, J = 6.6 Hz) CH₃, 2.97 ppm (m, 2H) CH₂; 3.50 ppm (d, 1H, J = 10.2 Hz) ½ CH₂; 3.92 ppm (d, 1H, J = 2.4 Hz) CH; 3.99 ppm (m, 1H) ½ CH_{2;} 4.41 ppm (d, 1H, J = 2.4 Hz) CH; 4.69 ppm (q, 1H, J = 6.6 Hz) CH; 7.05 ppm (t, 2H, J = 9.0 Hz) 2 x CH; 7.33 ppm (dd, 2H, J¹= 2.1 Hz, j² = 5.7 Hz) 2 x CH; 7.40 ppm (s, 2H) 2 x CH, 7.85 ppm (s, 1H) CH.
The NMR-spectrum of the product is shown in Figure 2. The integrals and the type of coupling of the signals at 3,9 and 4,4 ppm are characteristic for the hydrogenation product (protons in the morpholino ring). The absence of a signal at 5,15ppm (characteristic for the starting material) indicates the essential completeness of the reaction.

### Example 3

88.8 g (4S,4aS,5aR,12aS)-4,7-bis(dimethylamino)-9-nitro-1,4,4a,5,5a,6,11,12a-octahydro-3,10,12,12a-tetrahydroxy-1,11-dioxo-2-naphthacencarboxamide were dissolved in 2.7 L methanol and 40 mL concentrated hydrochloric acid. 30.2 g of catalyst (10 % palladium on carbon wetted with 50 % water, BASF type #286063) was added. A mixture of 95 vol.% N₂ and 5 vol.% H₂ (available from Linde Gas) was passed through the suspension for 6.5 hours at a flow rate of 80 L/h using a glass filter candle. The temperature was 20 to 25 °C and the overpressure was approx. 130 mbar. After HPLC had shown that the substrate had completely reacted, the catalyst was removed by filtration. The solution was concentrated *in vacuo.* 1.4 L water was given to the resultant liquid and the product was crystallized with the help of 110 mL 5% ammonia solution. The crystals were isolated using a Büchner funnel and dried at *35 °C in vacuo.* 77.2 g (4S,4aS,5aR,12aS)-9-amino-4,7-bis(dimethylamino)-1,4,4a,5,5a,6,11,12a-octahydro-3,10,12,12a-tetrahydroxy-1,11-dioxo-2-naphthacencarboxamide hydrochloride dihydrate were obtained.

The purity of the product was 99.3 % as determined using HPLC. The unreduced starting compound (4S,4aS,5aR,12aS)-4,7-bis(dimethylamino)-9-nitro-1,4,4a,5,5a,6,11,12a-octahydro-3,10,12,12a-tetrahydroxy-1,11-dioxo-2-naphthacencarboxamide runs at about 10.3 min in this assay and is barely detectable with a peak area of below 0.1 %. For sake of comparability, the peak areas at 6.856, 7.072 and 7.663 are 0.14%, 0.18% and 0.11 %, respectively. Thus reduction of the nitro compound to the corresponding amine was thus essentially complete.

## Claims

1. A process for the reaction of a compound with hydrogen wherein the reaction is conducted using a hydrogen-containing gas comprising up to 10 vol.% hydrogen and at least 90 vol.% of an inert gas and wherein the compound to be reacted with hydrogen is provided in a liquid phase, and wherein the reaction is a hydrogenation reaction and the compound to be reacted with hydrogen contains a double bond or a triple bond selected from the group consisting of

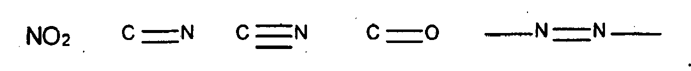

2. The process according to claim 1, wherein the compound to be reacted with hydrogen is a liquid or is dissolved, suspended or emulsified in the liquid phase.

3. The process according to claim 1 or 2, wherein the hydrogen-containing gas is passed through the liquid phase.

4. The process according to any one of claims 1 to 3, wherein the reaction is conducted in the presence of a homogeneous or heterogeneous catalyst.

5. The process according to claim 4, wherein the catalyst comprises a platinum group metal or nickel.

6. The process according to any one of claims 1 to 5, wherein the inert gas is nitrogen.

7. The process according to any of claims 1 to 6, wherein the gas comprises 0.1 vol.% to 10 vol.% hydrogen and 90 vol.% to 99.9 vol.% of an inert gas; preferably 1 vol.% to 7 vol.% hydrogen and 93 vol.% to 99 vol.% of an inert gas; more preferably 2 vol.% to 6 vol.% hydrogen and 94 vol.% to 98 vol.% of an inert gas; and even more preferably 5 vol.% hydrogen and 95 vol.% of an inert gas.

8. A process for the reaction of a compound with hydrogen wherein the reaction is conducted using a hydrogen-containing gas comprising up to 10 vol.% hydrogen and at least 90 vol.% of an inert gas and wherein the compound to be reacted with hydrogen is provided in a liquid phase, wherein the reaction is a hydrogenolysis reaction.

9. The process according to claim 8, wherein the compound has a moiety selected from the group consisting of

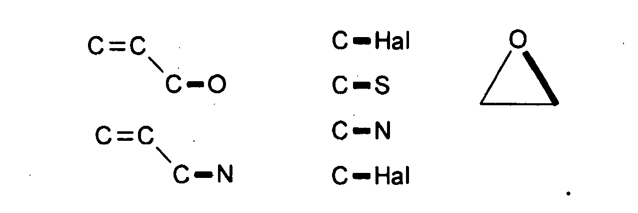

10. Use of a hydrogen-containing gas comprising up to 10 vol.% hydrogen and at least 90 vol.% of an inert gas for the catalytic hydrogenation as defined in claims 4-7 or hydrogenolysis as defined in claims 8 and 9 of an organic compound susceptible to catalytic hydrogenation or hydrogenolysis, wherein the organic compound to be used as the substrate for catalytic hydrogenation or hydrogenolysis is provided in a liquid phase.

11. Use according to claim 10, wherein the substrate for catalytic hydrogenation contains a double bond or a triple bond selected from the group consisting of

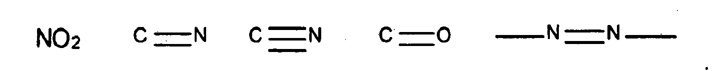

12. Use according to claim 10, wherein the substrate for catalytic hydrogenolysis contains a moiety selected from the group consisting of

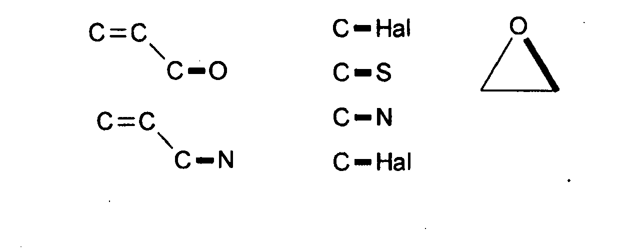

## Patentansprüche

1. Verfahren für die Reaktion einer Verbindung mit Wasserstoff, wobei die Reaktion unter Verwendung eines Wasserstoff-enthaltenden Gases, umfassend bis zu 10 Vol.-% Wasserstoff und mindestens 90 Vol.-% eines inerten Gases, durchgeführt wird, und wobei die mit Wasserstoff zu reagierende Verbindung in einer flüssigen Phase zur Verfügung gestellt wird, und wobei die Reaktion eine Hydrierungsreaktion ist und die mit Wasserstoff zu reagierende Verbindung eine Doppelbindung oder eine Dreifachbindung, ausgewählt aus der Gruppe bestehend aus 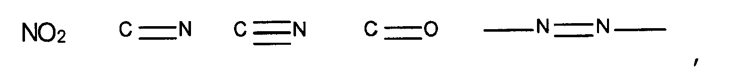 enthält.

2. Das Verfahren nach Anspruch 1, wobei die mit Wasserstoff zu reagierende Verbindung eine Flüssigkeit ist, oder in der flüssigen Phase aufgelöst, suspendiert oder emulgiert ist.

3. Das Verfahren nach Anspruch 1 oder 2, wobei das Wasserstoff-enthaltende Gas durch die flüssige Phase geleitet wird.

4. Das Verfahren nach irgendeinem der Ansprüche 1 bis 3, wobei die Reaktion in der Gegenwart eines homogenen oder heterogenen Katalysators durchgeführt wird.

5. Das Verfahren nach Anspruch 4, wobei der Katalysator ein Platingruppenmetall oder Nickel umfasst.

6. Das Verfahren nach irgendeinem der Ansprüche 1 bis 5, wobei das inerte Gas Stickstoff ist.

7. Das Verfahren nach irgendeinem der Ansprüche 1 bis 6, wobei das Gas 0,1 Vol.-% bis 10 Vol.-% Wasserstoff und 90 Vol.-% bis 99,9 Vol.-% eines inerten Gases, bevorzugt 1 Vol.-% bis 7 Vol.-% Wasserstoff und 93 Vol.-% bis 99 Vol.-% eines inerten Gases; stärker bevorzugt 2 Vol.-% bis 6 Vol.-% Wasserstoff und 94 Vol.-% bis 98 Vol.-% eines inerten Gases; und noch stärker bevorzugt 5 Vol.-% Wasserstoff und 95 Vol.-% eines inerten Gases, umfasst.

8. Verfahren für die Reaktion einer Verbindung mit Wasserstoff, wobei die Reaktion unter Verwendung eines Wasserstoff-enthaltenden Gases umfassend bis zu 10 Vol.-% Wasserstoff und mindestens 90 Vol.-% eines inerten Gases, durchgeführt wird und wobei die mit Wasserstoff zu reagierende Verbindung in einer flüssigen Phase bereitgestellt wird, wobei die Reaktion eine Hydrogenolyse-Reaktion ist.

9. Das Verfahren nach Anspruch 8, wobei die Verbindung eine Einheit aufweist, ausgewählt aus der Gruppe bestehend aus:

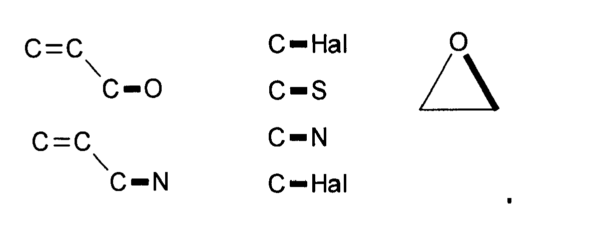

10. Verwendung eines Wasserstoff-enthaltenden Gases umfassend bis zu 10 Vol.-% Wasserstoff und mindestens 90 Vol.-% eines inerten Gases für die katalytische Hydrierung wie in Ansprüchen 4 bis 7 definiert oder die Hydrogenolyse wie in den Ansprüchen 8 und 9 definiert von einer organischen Verbindung die für eine katalytische Hydrierung oder Hydrogenolyse empfänglich ist, wobei die organische Verbindung, die als das Substrat für die katalytische Hydrierung oder Hydrogenolyse verwendet wird in einer flüssigen Phase bereitgestellt wird.

11. Verwendung nach Anspruch 10, wobei das Substrat für die katalytische Hydrierung eine Doppelbindung oder eine Dreifachbindung, ausgewählt aus der Gruppe bestehend aus 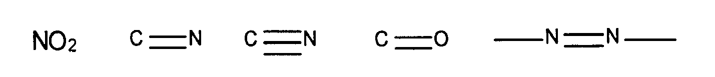 enthält.

12. Verwendung nach Anspruch 10, wobei das Substrat für die katalytische Hydrogenolyse eine Einheit enthält, die ausgewählt ist aus der Gruppe bestehend aus:

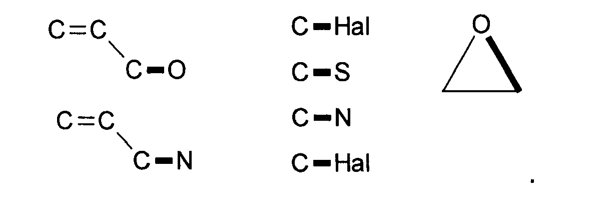

## Revendications

1. Procédé pour la réaction d'un composé avec de l'hydrogène, dans lequel la réaction est menée en utilisant un gaz contenant de l'hydrogène comprenant jusqu'à 10 % en vol. d'hydrogène et au moins 90 % en vol. d'un gaz inerte et dans lequel le composé à faire réagir avec l'hydrogène est fourni dans une phase liquide, et dans lequel la réaction est une réaction d'hydrogénation et le composé à faire réagir avec l'hydrogène contient une double liaison ou une triple liaison choisie dans le groupe constitué par

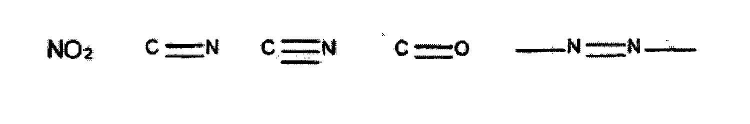

2. Procédé selon la revendication 1, dans lequel le composé à faire réagir avec de l'hydrogène est un liquide ou est dissous, mis en suspension ou émulsionné dans la phase liquide.

3. Procédé selon la revendication 1 ou 2, dans lequel le gaz contenant de l'hydrogène est fait passer à travers la phase liquide.

4. Procédé selon l'une quelconque des revendications 1 à 3, dans lequel la réaction est menée en présence d'un catalyseur homogène ou hétérogène.

5. Procédé selon la revendication 4, dans lequel le catalyseur comprend un métal du groupe du platine ou du nickel.

6. Procédé selon l'une quelconque des revendications 1 à 5, dans lequel le gaz inerte est l'azote.

7. Procédé selon l'une quelconque des revendications 1 à 6, dans lequel le gaz comprend 0,1 % en vol. à 10 % en vol. d'hydrogène et 90 % en vol. à 99,9 % en vol. d'un gaz inerte ; de préférence 1 % en vol. à 7 % en vol. d'hydrogène et 93 % en vol. à 99 % en vol. d'un gaz inerte ; plus préférablement 2 % en vol. à 6 % en vol. d'hydrogène et 94 % en vol. à 98 % en vol. d'un gaz inerte ; et encore plus préférablement 5 % en vol. d'hydrogène et 95 % en vol. d'un gaz inerte.

8. Procédé pour la réaction d'un composé avec de l'hydrogène, dans lequel la réaction est menée en utilisant un gaz contenant de l'hydrogène comprenant jusqu'à 10 % en vol. d'hydrogène et au moins 90 % en vol. d'un gaz inerte et dans lequel le composé à faire réagir avec l'hydrogène est fourni dans une phase liquide, dans lequel la réaction est une réaction d'hydrogénolyse.

9. Procédé selon la revendication 8, dans lequel le composé a une fraction choisie dans le groupe constitué par

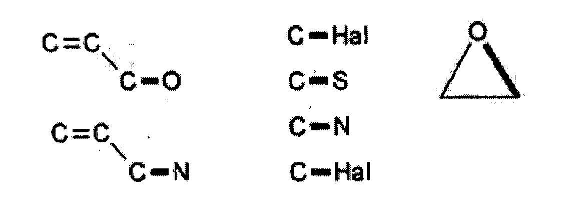

10. Utilisation d'un gaz contenant de l'hydrogène, comprenant jusqu'à 10 % en vol. d'hydrogène et au moins 90 % en vol. d'un gaz inerte pour l'hydrogénation catalytique telle que définie selon les revendications 4 à 7 ou l'hydrogénolyse telle que définie selon les revendications 8 et 9 d'un composé organique sensible à une hydrogénation catalytique ou une hydrogénolyse, dans laquelle le composé organique à utiliser en tant que substrat pour l'hydrogénation catalytique ou l'hydrogénolyse est fourni dans une phase liquide.

11. Utilisation selon la revendication 10, dans laquelle le substrat pour l'hydrogénation catalytique contient une double liaison ou une triple liaison choisie dans le groupe constitué par

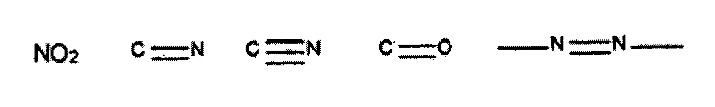

12. Utilisation selon la revendication 10, dans laquelle le substrat pour l'hydrogénolyse catalytique contient une fraction choisie dans le groupe constitué par
